# EUROPÄISCHE PATENTSCHRIFT

(11) **EP 2 344 620 B1**
(45) Veröffentlichungstag und Bekanntmachung des Hinweises auf die Patenterteilung: **28.05.2014**
(21) Anmeldenummer: 09740646.6
(22) Anmeldetag: 01.10.2009
(51) Int. Cl.: C12M 1/00, C12M 3/00, C12M 1/34

(54) **Kultur-/Expositionsvorrichtung für Zell- und/oder Bakterienkulturen**
Culture/exposure device for cell and/or bacteria cultures
Dispositif de culture/d'exposition pour des cultures de cellules et/ou de bactéries

(30) Priorität: 07.04.2009 DE 102009016364; 11.11.2008 DE 102008056685; 11.11.2008 DE 102008056763; 11.11.2008 DE 102008056684; 11.11.2008 DE 102008056686; 06.10.2008 DE 102008050080; 06.10.2008 DE 102008050076; 06.10.2008 DE 102008050077; 06.10.2008 DE 102008050079
(43) Veröffentlichungstag der Anmeldung: 20.07.2011
(73) Patentinhaber: Mohr, Ulrich, 30559 Hannover (DE)
(72) Erfinder: AUFDERHEIDE, Michaela, 30559 Hannover (DE); HALTER, Beat, CH-9100 Herisau (CH)
(74) Vertreter: Arat, Dogan
(86) Internationale Anmeldenummer: PCT/EP2009/007054
(87) Internationale Veröffentlichungsnummer: WO 2010/040473

(56) Entgegenhaltungen:
- EP-A1- 1 431 746
- EP-A2- 1 174 496
- WO-A1-2007/085621
- DE-A1- 10 014 057
- DE-A1- 10 211 324
- DE-A1- 19 526 533
- US-A1- 2006 024 822

## Beschreibung

Die Erfindung betrifft eine Kultur-/Expositionsvorrichtung der im Oberbegriff des Anspruches 1 genannten Art, insbesondere für Zell- und/oder Bakterienkulturen.

### Stand der Technik

Derartige Kultur-/Expositionsvorrichtungen sind allgemein bekannt und dienen beispielsweise dazu, eine Zellkultur mit einer Testatmosphäre zu beaufschlagen und zu kultivieren, um festzustellen, welche Auswirkungen die Testatmosphäre auf die Zellkultur hat. Insbesondere kann untersucht werden, welche Auswirkungen beispielsweise Gase, Aerosole und/oder partikuläre Wirkstoffe auf die Zell- und Bakterienkulturen haben.

Kultur-/Expositionsvorrichtungen der betreffenden Art sind durch EP 1 049 765 B1 und DE 102 11 324 A1 bekannt. Sie weisen wenigstens zwei Aufnahmen für Kulturbehälter und eine Strömungsführung zum Beaufschlagen der Kulturbehälter mit einer Testatmosphäre auf. Von der Strömungsführung zu den Kulturbehältern verlaufen Strömungskanäle, durch die die Testatmosphäre zu in den Kulturbehältern aufgenommenen Zellkufturen geleitet wird. Nachdem die Zellkulturen mit der Testatmosphäre beaufschlagt worden sind, werden sie kultiviert, nach Abschluss der Kultivierung entnommen und untersucht. Auf diese Weise lassen sich Rückschlüsse ziehen, ob und in welcher Hinsicht die Testatmosphäre Auswirkungen auf die Kulturen hat.

Weiterhin ist aus der DE 102 11 324 A1 eine Kultur-/Expositionsvorrichtung zum Aufnehmen von Kulturen mit einer Einrichtung zum Beaufschlagen der aufgenommenen Kultur mit einem gasförmigen Medium bekannt, die eine mechanische Strömungsführung mit einem Eingang zum Einleiten des gasförmigen Mediums in die Strömungsführung und einer oberhalb der Oberfläche der Kultur mündenden Ausgangsmundung umfasst, wobei die Strömungsführung das gasförmige Medium zwangsführt.

Die DE 100 14 057 A1 betrifft eine Expositionsvorrichtung zum Versorgen einer in einem Kulturgefäss aufgenommenen Kultur mit einem flüssigen Medium, welches eine Beaufschlagungsvorrichtung aufweist.

Die US 2006/0024822 A1 offenbart ein System zum Kultivieren von Zellen, welches einen Bioreaktor, in welchem die Zellen angeordnet sind, eine Pumpe zum Zuführen von Gas in den Bioreaktor sowie ein elektromagnetisches Modul umfasst, mittels welchem ein elektromagnetisches Feld angelegt werden kann.

Die DE 195 26 533 A1 offenbart eine Expositionsvorrichtung für mit gas- und/oder partikelförmigen Beimengungen angereicherte Gasgemische auf lebende Zellkulturen, bei der das definiert angereicherte Gasgemisch über ein Zuleitungssystem direkt an mindestens einen Träger für die lebenden Zellkulturen führbar ist, wobei das Nährmedium über ein weiteres Zuleitungssystem von unten durch eine permeable Membran an die Zellkulturen führbar ist und das Zuleitungssystem für das angereicherte Gasgemisch so ausgebildet ist, dass es von oben auf die Zellkulturen exponierend wirkt.

Die in der EP 1 431 746 A1 offenbarte Vorrichtung umfasst einen Rauch emittierenden Abschnitt, welcher Partikel enthaltenden Rauch emittiert, eine Verdünnungseinrichtung zur Verdünnung des Rauchs, eine Einheit, die der Verdünnungseinrichtung saubere Luft zuführt, zumindest eine Kammer, die eine zellhaftende Oberfläche aufweist und in die der durch die Verdünnungseinrichtung verdünnte Rauch eingeführt wird, und eine Absaugeinrichtung zum Absaugen des Rauches aus der Kammer (10) und der Verdünnungseinrichtung.

### Aufgabe

Der Erfindung liegt die Aufgabe zugrunde, eine Kultur-/Expositionsvorrichtung der im Oberbegriff des Anspruches 1 genannten Art, insbesondere für Zell- und/oder Bakterienkulturen, anzugeben, bei der die Genauigkeit bzw. Reproduzierbarkeit der Untersuchungsergebnisse verbessert ist und die Untersuchungsmöglichkeiten erweitert sind.

### Lösung der Aufgabe

Diese Aufgabe wird durch die in Anspruch 1 angegebene Erfindung gelöst.

Ein erster Grundgedanke der vorliegenden Erfindung besteht darin, die gesamte Vorrichtung modular aufzubauen, sodass sie leicht zu betätigen ist und leicht auseinander genommen werden kann. Die einzelnen Module sind so ausgestaltet, dass sie auch durch andere Module ersetzt werden können. Des Weiteren ist aber auch daran gedacht, die Module intramodular austauschbar auszugestalten, das heißt, wesentliche Elemente der einzelnen Module sind wieder durch andere Untermodule austauschbar. Auf diese Weise wird eine sehr flexibel einsetzbare Vorrichtung geschaffen.

Ein Gedanke der Erfindung in dieser Hinsicht besteht darin, dass sich die Untersuchungsmöglichkeiten im Hinblick auf Zell- und/oder Bakterienkulturen, die nachfolgend auch kurz als Kulturen bezeichnet werden, dadurch erweitern lassen, dass die Testatmosphäre, mit der die Kulturen beaufschlagt werden, entsprechend den jeweiligen Anforderungen vorbehandelt wird. Die Erfindung sieht hierzu vor, dass der Einlass an einem lösbar mit dem Grundkörper verbundenen Vorbereitungs-Modul gebildet ist. Erfindungsgemäss ist das Vorbereitungs-Modul also austauschbar ausgebildet, so dass entsprechend der jeweils gewünschten Untersuchung ein geeignetes Vorbereitungs-Modul verwendet werden kann.

Beispielsweise und insbesondere ist es möglich, ein Vorbereitungs-Modul zu verwenden, bei dem eine Vorbehandlung der Testatmosphäre unterbleibt, die Testatmosphäre von dem Einlass zu dem Kulturbehälter durchgeleitet wird.

Wenn demgegenüber gewünscht ist, in möglichst hohem Masse in der Testatmosphäre enthaltene Partikel auf den Kulturen abzuscheiden, so ist es zweckmässig, wenn die Partikel mittels eines Vorbereitungs-Moduls elektrostatisch aufgeladen werden, bevor sie dem Kulturbehälter zugeleitet werden. In diesem Falle kann beispielsweise das Vorbereitungs-Modul ohne Vorbehandlung der Testatmosphäre gegen ein solches mit elektrostatischer Aufladung ausgetauscht werden.

Erfindungsgemäss sind somit die Untersuchungsmöglichkeiten im Hinblick auf Zell- und/oder Bakterienkulturen wesentlich erweitert.

Eine Weiterbildung der Erfindung sieht werkzeuglos lösbare Verbindungsmittel zum Verbinden des Vorbereitungs-Moduls mit dem Grundkörper vor. Bei dieser Ausführungsform ist das Vorbereitungs-Modul werkzeuglos austauschbar, so dass der Austausch besonders schnell und einfach vonstatten geht.

Eine zweckmässige Weiterbildung der vorgenannten Ausführungsform sieht vor, dass die Verbindungsmittel einen Formschluss zwischen dem Vorbereitungs-Modul und dem Grundkörper herstellen. Auf diese Weise ist das Vorbereitungs-Modul sicher an dem Grundkörper gehalten.

Eine Weiterbildung der Ausführungsform mit dem Vorbereitungs-Modul sieht vor, dass mit dem Grundkörper lösbar ein Sockel-Modul zum Anlegen eines elektrostatischen Feldes an dem Kulturbehälter verbunden ist.

Ein weiterer Teil der Erfindung löst die zugrunde liegende Aufgabe dadurch, dass der Grundkörper aus einem Aerosolführungs-Modul und einem Probenaufnahme-Modul besteht und dass Aerosolführungs-Modul und Probenaufnahme-Modul an einer Führung relativ zueinander geführt sind und durch eine Antriebseinrichtung zwischen einer Schliessstellung, in der die Strömungsführung strömungstechnisch mit dem Kulturbehälter in Verbindung steht, und einer Offenstellung, in der die Strömungsführung strömungstechnisch von dem Kulturbehälter getrennt ist, relativ zueinander bewegbar sind.

Auf diese Weise ist erreicht, dass die strömungstechnische Trennung des Aerosolführungs-Moduls vom Probenaufnahme-Modul auf einfache Weise erfolgen kann, wodurch die Kultur-/Expositionsvorrichtung zudem sicher bedienbar ist. Ferner ist dadurch erreicht, dass die benötigte Zeit für das strömungstechnische Trennen von Aerosolführungs-Modul und Probenaufnahme-Modul verkürzt ist, da die Erfindung auf zusätzliche Verriegelungs-Module, wie z.B. Schrauben, Klemmen und dergleichen, zum Fixieren der Schliessstellung verzichtet.

Zudem ist die strömungstechnische Verbindung von Aerosolführungs-Modul und Probenaufnahme-Modul durch die Führung des Aerosolführungs-Moduls und des Probenaufnahme-Moduls präzisiert.

Die relative Bewegung des Aerosolführungs-Moduls und des Probenaufnahme-Moduls zueinander kann auf unterschiedliche Weise erfolgen. Erfindungsgemäss ist es dazu möglich, sowohl Aerosolführungs-Modul als auch Probenaufnahme-Modul gleichzeitig oder nacheinander zu bewegen. Ferner ist es möglich, lediglich das Aerosolführungs-Modul relativ zum Probenaufnahme-Modul zu bewegen, während das Probenaufnahme-Modul bewegbar bleibt. Darüber hinaus ist ebenfalls eine umgekehrte Bewegungskette möglich, so dass das Probenaufnahme-Modul bewegt wird, während das Aerosolführungs-Modul feststeht.

Eine Weiterbildung der Erfindung besteht darin, dass die Antriebseinrichtung manuell betätigbar ist. Dadurch ergibt sich der Vorteil einer kostengünstigen Realisierung und Nutzung der Kultur-/Expositionsvorrichtung. Ferner ist diese dadurch unabhängig von insbesondere elektrischer Energie.

Gemäss einer weiteren Weiterbildung der Erfindung weist die Führung wenigstens eine erste Linearführung zur linearen Führung des Aerosolführungs-Moduls und des Probenaufnahme-Moduls relativ zueinander zwischen der Schliessstellung und der Offenstellung auf. Dadurch wird erreicht, dass das Zusammenführen von Aerosolführungs-Modul und Probenaufnahme-Modul präzise, schnell und sicher erfolgt.

Darüber hinaus ist eine weitere Weiterbildung der Erfindung dadurch gekennzeichnet, dass die erste Linearführung zur Erzeugung einer selbsttätigen bzw. nahezu selbsttätigen Bewegung des Aerosolführungs-Moduls in die Offenstellung oder in die Schliessstellung wenigstens eine Feder, insbesondere eine Spiralfeder, aufweist. Die Feder wird in einer Bewegungsrichtung gespannt, wodurch sie in entgegengesetzter Richtung die gespeicherte Energie als kinetische Energie wieder abgeben kann.

Dadurch ist es möglich, die Feder beispielsweise in einer Bewegung zur Erreichung der Schliessstellung zu spannen, um die gespeicherte Energie für ein selbsttätiges Erreichen der Offenstellung zu nutzen. Die Ausprägung der Feder als Zug- oder Druckfeder hängt dabei von der konstruktiven Anordnung der Feder wie auch der zu unterstützenden Bewegungsrichtung ab. Daher ist es möglich, die Unterstützung durch die Feder für eine Bewegung zur Erzielung der Offenstellung oder der Schliessstellung zu nutzen. Diese Unterstützung ist für die Bewegung des Aerosolführungs-Moduls wie auch für die Bewegung des Probenaufnahme-Moduls möglich, indem wenigstens eine Feder entsprechend dem Aerosolführungs- bzw. Probenaufnahme-Modul zugeordnet wird. Die Feder ermöglicht es nicht nur, eine Bewegungsrichtung des Aerosolführungs-Moduls und/oder des Probenaufnahme-Moduls zu unterstützen, sondern auch für die Bewegung des Aerosolführungs- und/oder Probenaufnahme-Moduls beispielsweise Seile zu verwenden, die durch die Feder unter Spannung gehalten werden können.

Darüber hinaus sieht die Weiterbildung der Erfindung vor, dass die Antriebseinrichtung wenigstens ein Getriebe aufweist zur Umformung einer Eingangsbewegung in eine Antriebsbewegung zum relativen Bewegen des Aerosolführungs-Moduls zum Probenaufnahme-Modul und/oder zur Umformung einer Eingangsbewegung in eine Antriebsbewegung zum relativen Bewegen des Aerosolführungs-Moduls zum Probenaufnahme-Modul und/oder zur Umformung einer Eingangsbewegung in eine Antriebsbewegung zum relativen Bewegen des Probenaufnahme-Moduls zum Aerosolführungs-Modul. Dadurch wird erreicht, dass die benötigten Kräfte zur Erzeugung der relativen Bewegung des Aerosolführungs-Moduls zum Probenaufnahme-Modul bzw. des Probenaufnahme-Moduls zum Aerosolführungs-Modul verringert sind.

Eine weitere Weiterbildung der Erfindung ist dadurch gegeben, dass das Getriebe wenigstens ein seil-, band- oder kettenförmiges Zugmittel aufweist zur Weiterleitung der Eingangsbewegung als eine Antriebsbewegung zum relativen Bewegen des Aerosolführungs-Moduls zum Probenaufnahme-Modul und/oder zum relativen Bewegen des Probenaufnahme-Moduls zum Aerosolführungs-Modul. Auf diese Weise wird erreicht, dass die bewegten trägen Massen des Getriebes gering gehalten werden, wodurch der Wirkungsgrad des Getriebes gesteigert ist. Ferner ist es dadurch möglich, die Aufnahme der Eingangsbewegung wie auch die Abgabe der Antriebsbewegung räumlich flexibel zu gestalten, wodurch Kostenvorteile erreicht sind.

Zudem besteht eine andere Weiterbildung der Erfindung darin, dass das Getriebe die Eingangsbewegung in eine Senkbewegung des Aerosolführungs-Moduls zum Probenaufnahme-Modul umformt, insbesondere in eine vertikale oder im wesentlichen vertikale Senkbewegung.

Ferner ist eine Weiterbildung der Erfindung dadurch gekennzeichnet, dass die Antriebseinrichtung wenigstens ein Betätigungsmittel aufweist zur Erzeugung der Eingangsbewegung. Das Betätigungsmittel dient dazu, die Bewegung des Aerosolführungs-Moduls bzw. Probenaufnahme-Moduls einzuleiten. Als Betätigungsmittel können verschiedene Mittel verwendet werden, die z.B. elektrisch, hydraulisch oder pneumatisch betrieben werden. Zudem sind manuell betätigbare Betätigungsmittel, wie es beispielsweise eine Drehkurbel oder ein Hebelmechanismus sein kann, eine insbesondere im medizinischen Bereich bevorzugte Ausführungsform, um den dort geltenden hohen hygienischen Anforderungen auf möglichst einfache und kostengünstige Weise gerecht werden zu können.

Darüber hinaus sieht eine Weiterbildung der Erfindung vor, dass die Führung eine zweite Linearführung aufweist, zur linearen Führung des Probenaufnahme-Moduls orthogonal bzw. im wesentlichen orthogonal zur Führungsrichtung der ersten Linearführung, insbesondere zur horizontalen oder im wesentlichen horizontalen Führung des Probenaufnahme-Moduls. Eine zweite Linearführung bewirkt eine einfachere Handhabung der Kultur-/Expositionsvorrichtung, da das Aerosolführungs-Modul und das Probenaufnahme-Modul einfacher zueinander positionierbar sind. So ist z.B. ein Wechsel des Kulturbehälters bzw. der Kulturbehälter in einem kürzeren Zeitintervall möglich.

Ein weiterer Gedanke der vorliegenden Erfindung bezieht sich darauf, dass die Strömungsöffnung eine Verteilungsöffnung oder einen Verteilungsraum aufweist, von der bzw. von dem Strömungskanäle im wesentlichen gleicher Länge zu den einzelnen Kulturbehältern führen. Dementsprechend ist sichergestellt, dass Teilströmungen der Testatmosphäre, die den einzelnen Kulturbehältern zugeleitet werden, zwischen der Verteilungsöffnung bzw. dem Verteilungsraum und dem jeweiligen Kulturbehälter im wesentlichen den gleichen Strömungsweg zurücklegen. Auf diese Weise ist verhindert, dass sich die Zusammensetzung der Testatmosphäre, beispielsweise von Rauch, aufgrund unterschiedlich langer Strömungswege zu den einzelnen Kulturbehältern in unterschiedlichem Masse ändert, so dass die Untersuchungsergebnisse verfälscht werden.

Die Erfindung stellt damit mit einfachen Mitteln eine Kultur-/Expositionsvorrichtung zur Verfügung, bei der die Genauigkeit bzw. Reproduzierbarkeit der Untersuchungsergebnisse verbessert ist.

Bei der Testatmosphäre kann es sich erfindungsgemäss insbesondere um ein gasförmiges Medium handeln, das Partikel trägt, beispielsweise ein Aeorosol. Erfindungsgemäss können jedoch auch andere Testatmosphären verwendet werden.

Eine Weiterbildung der Erfindung sieht vor, dass die Aufnahmen für die Kulturbehälter entlang des Umfanges eines Kreises angeordnet sind, vorzugsweise in Umfangrichtung im wesentlichen äquidistant. Bei dieser Ausführungsform ist die Ausbildung von Strömungskanälen im wesentlichen gleicher Länge vereinfacht.

Eine Weiterbildung der vorgenannten Ausführungsform sieht vor, dass eine Längsachse des ausgangsseitigen Endes der Strömungsführung im wesentlichen mit dem Mittelpunkt des Kreises zusammenfällt.

Gemäss einer anderen Weiterbildung der Erfindung verlaufen die Strömungskanäle von der Längsachse des auslassseitigen Endes der Strömungsführung geneigt zu den Kulturbehältern. Bei dieser Ausführungsform ergibt sich ein besonders einfacher und kompakter Aufbau der Kultur-/Expositionsvorrichtung, die nachfolgend auch kurz als Vorrichtung bezeichnet wird.

Die Strömungskanäle können auf beliebige geeignete Weise gebildet sein, beispielsweise durch Schläuche oder Rohrleitungen. Um den Aufbau der erfindungsgemässen Vorrichtung besonders kompakt und widerstandsfähig zu gestalten, sieht eine andere Weiterbildung der Erfindung vor, dass der Grundkörper einen Strömungskanalblock aufweiset, in dem die Verteilungsöffnung bzw. der Verteilungsraum und die Strömungskanäle gebildet sind.

Eine ausserordentlich vorteilhafte Weiterbildung der Erfindung sieht vor, dass die Längsachse des Verteilungsraumes bzw. der Verteilungsöffnung mit der Längsachse des jeweiligen Strömungskanals einen Winkel bildet, der kleiner, vorzugsweise wesentlich kleiner, als 90° ist. Auf diese Weise ergibt sich ein besonders ungestörter Strömungsverlauf. Ausserdem ist verhindert, dass sich an Ecken der Strömungsführung in der Testatmosphäre enthaltene Partikel übermässig ablagern.

Der Erfindung liegt der weitere Gedanke zugrunde, die mit der erfindungsgemässen Kultur-/Expositionsvorrichtung erzielten Untersuchungsergebnisse dadurch zu verbessern, dass die Temperierung des Kulturbehälters verbessert wird. Zu diesem Zweck sieht die Erfindung vor, dass die Aufnahme in dem Strömungsweg angeordnet ist, derart, dass der Kulturbehälter ein Strömungshindernis für das Temperierfluid bildet. Dadurch, dass der Kulturbehälter ein Strömungshindernis für das Temperierfluid bildet, wird der Kulturbehälter intensiv von dem Temperierfluid umströmt und damit besonders gleichmässig temperiert. Durch entsprechende Wahl der Temperatur der Temperierflüssigkeit lässt sich auf diese Weise der Kulturbehälter und damit eine darin aufgenommene Kultur besonders genau temperieren, um beispielsweise eine Kondensatbildung zu vermeiden. Dadurch, dass die Aufnahme im Strömungsweg des Temperierfluids angeordnet ist, sind hydrodynamische Kurzschlüsse des Temperierfluids zwischen Ein- und Auslass vermieden.

Erfindungsgemäss weist die Kammer eine im wesentlichen kreisförmig begrenzte Innenwandung auf. Auf diese Weise ergeben sich besonders günstige Strömungsverhältnisse des Temperierfluids in der Kammer, die für eine besonders gleichmässige Temperierung des Kulturbehälters oder der Kulturbehälter sorgen.

Um die Strömungsverhältnisse des Temperierfluids weiter zu verbessern, sieht eine andere Weiterbildung der Erfindung vor, dass die Aufnahme eine im wesentlichen kreisförmig begrenzte Aussenwandung aufweist. Bei dieser Ausführungsform können Kulturbehälter mit kreisförmig begrenzter Aussenwandung verwendet werden, so dass sich besonders günstige Strömungsverhältnisse ergeben.

Andere vorteilhafte Weiterbildungen der Erfindung sehen vor, dass wenigstens zwei Einlässe und/oder wenigstens zwei Auslässe vorgesehen sind. Durchströmt bei diesen Ausführungsformen das Temperierfluid die Kammer, so bilden sich zwischen dem Einlass bzw. den Einlässen und dem Auslass bzw. den Auslässen wenigstens zwei Strömungswege aus, in denen jeweils eine Aufnahme für einen Kulturbehälter angeordnet ist, derart, dass jeder der Kulturbehälter ein Strömungshindernis für das Temperierfluid ermöglicht.

Die vorgenannte Ausführungsform ist besonders dann vorteilhaft, wenn wenigstens zwei Aufnahmen für Kulturbehälter vorgesehen sind, wie dies eine andere Weiterbildung der Erfindung vorsieht.

Eine vorteilhafte Weiterbildung der vorgenannten Ausführungsform sieht vor, dass die Aufnahmen entlang des Umfanges eines zu der Innenwandung der Kammer vorzugsweise konzentrischen Kreises angeordnet sind.

Grundsätzlich kann als Temperierfluid ein gasförmiges Fluid verwendet werden. Um auf einfache und genaue Weise eine Temperierung zu ermöglichen, sieht eine andere vorteilhafte Weiterbildung vor, dass das Temperierfluid eine Temperierfiüssigkeit ist.

Die Erfindung wird nachfolgend anhand der beigefügten Zeichnung näher erläutert, in der Ausführungsbeispiele erfindungsgemässer Vorrichtungen dargestellt sind. Dabei bilden alle in den Patentansprüchen beanspruchten, beschriebenen und in der Zeichnung dargestellten Merkmale für sich genommen sowie in beliebiger Kombination miteinander den Gegenstand der Erfindung, unabhängig von ihrer Zusammenfassung in den Patentsansprüchen und deren Rückbeziehung sowie unabhängig von ihrer Beschreibung bzw. Darstellung in der Zeichnung.

Es zeigt:
- Fig. 1: eine Explosionsdarstellung als erläuternde Darstellung eines Ausführungsbeispieles einer erfindungsgemässen Vorrichtung,
- Fig. 2: eine Perspektivansicht der Vorrichtung gemäss Fig. 1,
- Fig. 3: einen Vertikalschnitt durch die Vorrichtung gemäss Fig. 2,
- Fig. 4: eine teilweise geschnittene Perspektivansicht der Vorrichtung gemäss Fig. 2,
- Fig. 5: eine Explosionsdarstellung des Probenaufnahme-Moduls der Vorrichtung,
- Fig. 6: stark schematisiert eine Draufsicht auf die Kammer,
- Fig. 7: eine Perspektivansicht einer erfindungsgemässen Kultur-/Expositionsvorrichtung in der Offenstellung,
- Fig. 8: eine Perspektivansicht des Ausführungsbeispiels nach Figur 7 in der Schliessstellung,
- Fig. 9: einen Vertikalschnitt des Ausführungsbeispiels gemäss Fig. 7 in einer detailreduzierten Darstellungsweise und
- Fig. 10: die Unterseite des Ausführungsbeispiels nach Figur 7 in einer Perspektivansicht.

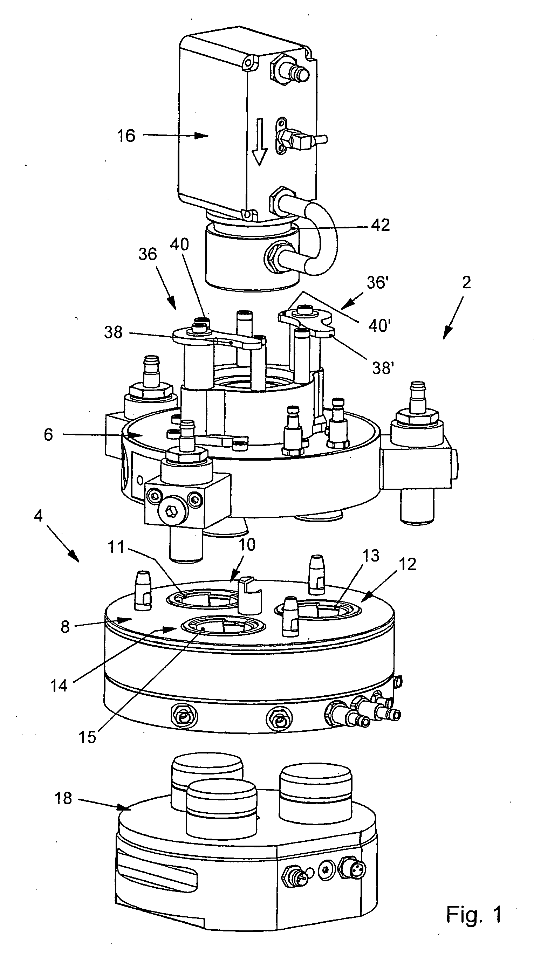

In Fig. 1 ist ein Ausführungsbeispiel einer nachfolgend kurz als Vorrichtung 2 bezeichneten Kultur/Expositionsvorrichtung gezeigt, die insbesondere für Zell- und/oder Bakterienkulturen geeignet ist. Die Vorrichtung 2 ist modulartig aufgebaut und besteht im vorliegend gezeigten Ausführungsbeispiel aus vier Modulen. Die Zell- und/oder Bakterienkulturen befinden sich in einem Probenaufnahme-Modul 8. Bei dem dargestellten Ausführungsbeispiel sind Aufnahmen 10, 12, 14 für Kulturbehälter 11, 13, 15 gebildet. In den Kulturbehältern 11, 13, 15 sind bei Benutzung der Vorrichtung bei diesem Ausführungsbeispiel Zellkulturen, beispielsweise in Form von Zellkulturinserten, aufgenommen, die gegebenenfalls kultiviert und mit einer Testatmosphäre beaufschlagt werden sollen.

Das Probenaufnahme-Modul 8 ist mit einem Aerosolführungs-Modul 6 verbunden, dem ein Vorbereitungs-Modul 16 aufsitzt.

Mittels diesem können in der Testatmosphäre enthaltene Partikel elektrostatisch aufgeladen werden. Um eine Abscheidung der elektrostatisch geladenen Partikel auf den Zellkulturen zu bewirken, ist mit dem Probenaufnahme-Modul 8 ein Sockel-Modul 18 verbindbar.

Des weiteren sind Verriegelungseinrichtungen 36, 36' vorgesehen, durch welches beispielsweise das Vorbereitungsmodul 16 mit dem Aerosolführungs-Modul 6 verriegelt werden kann.

Ein wesentlicher Teil der vorliegenden Erfindung besteht darin, dass einzelne Module als Ganzes durch Module mit anderen Eigenschaften ausgetauscht werden können. Ferner sind einzelne Module in sich nochmals modular aufgebaut und können so an verschiedene Bedürfnisse angepasst werden. Beim augenblicklichen Stand der Entwicklung werden nachfolgend bei den einzelnen Modulen auch die vorhandenen Austauschmöglichkeiten bzw. deren eigener modularer Aufbau beschrieben.

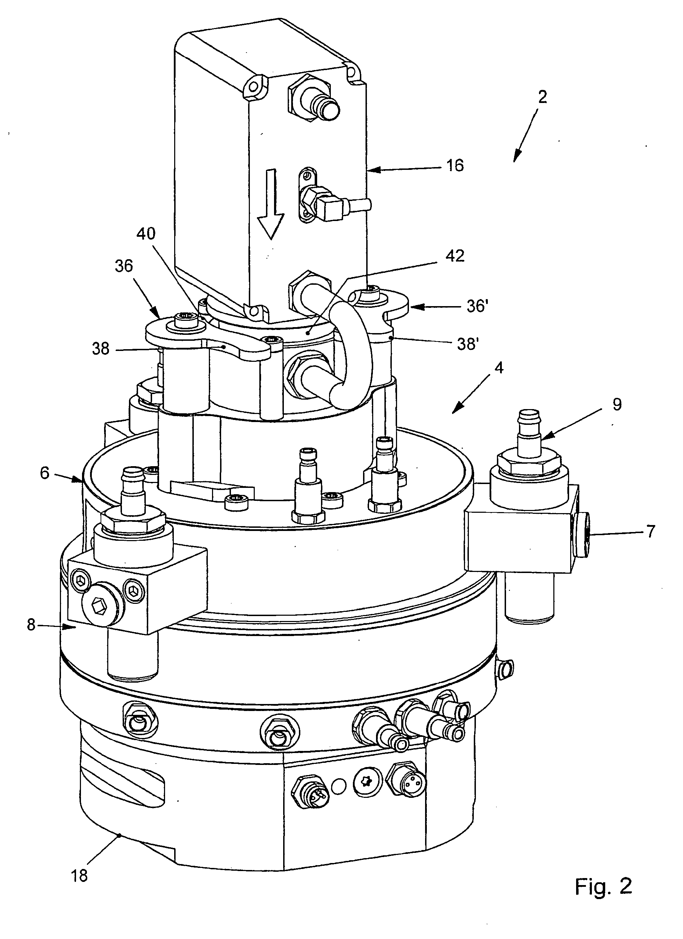

Fig. 2 zeigt eine Perspektivansicht der Vorrichtung 2, wobei das Aerosolführungs-Modul 6 mit dem Probenaufnahme-Modul 8 sowie das Vorbereitungs-Modul 16 mit dem Aerosolführungs-Modul 6 und das Sockel-Modul 18 mit dem Probenaufnahme-Modul 8 verbunden sind.

Das Vorbereitungs-Modul 16 kann beispielsweise als Auflader ausgebildet sein, wobei eine Testatmosphäre beispielsweise elektrostatisch aufgeladen wird. Dieses Modul könnte ersetzt werden durch einen Befeuchter, in welchem die Testatmosphäre befeuchtet wird oder durch einen einfachen Einlassadapter, wenn keine Vorbehandlung der Testatmosphäre gewünscht ist.

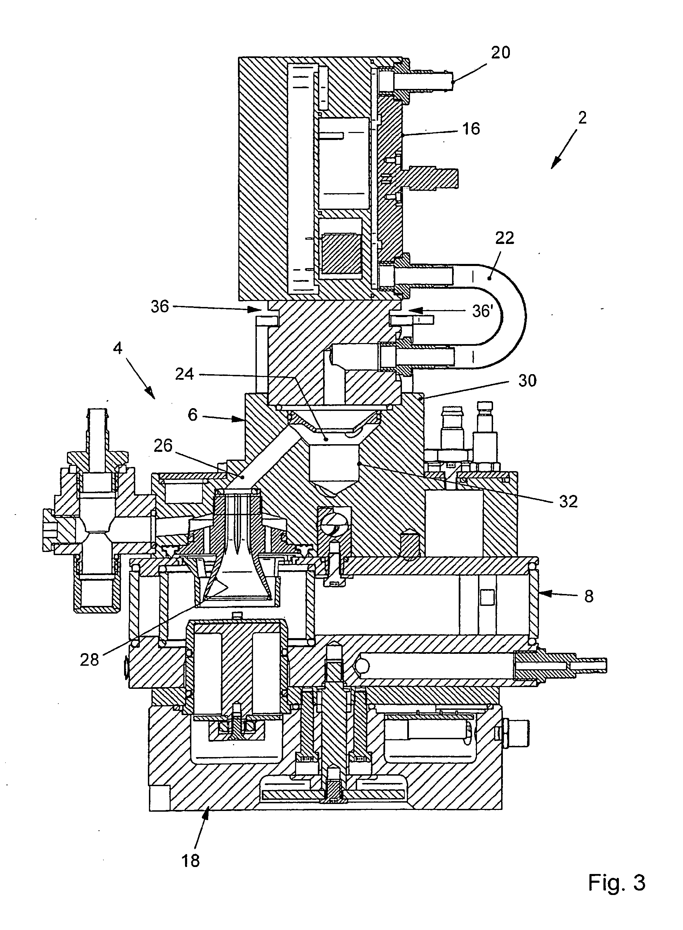

In dem in Figur 3 gezeigten Ausführungsbeispiel besitzt das Vorbereitungs-Modul 16 eine Einlassöffnung 20 über die eine Testatmosphäre, insbesondere ein mit Partikeln beaufschlagtes gasförmiges Medium, beispielsweise Rauch, in die Vorrichtung 2 eingeleitet werden kann.

Um die Testatmosphäre den Kulturbehältern 11, 13, 15 zuzuleiten, weist die Vorrichtung 2 eine Strömungsführung 22 auf, die das gasförmige Medium von dem Vorbereitungs-Modul 16 durch das Aerosolführungs-Modul 6 zu einem Verteilungsraum 24 leitet. Dieser befindet sich in einem Strömungskanalblock 30. Von ihm führen Strömungskanäle im wesentlichen gleicher Länge zu den einzelnen Kulturbehältern. Bei dem dargestellten Ausführungsbeispiel sind entsprechend den drei Aufnahmen 10, 12 und 14 und damit drei Kulturbehältern 11, 13, 15 drei Strömungskanäle vorgesehen, von denen im Schnitt lediglich ein Strömungskanal 26 erkennbar ist. Nachfolgend wird lediglich der Strömungskanal 26 näher erläutert. Die anderen beiden Strömungskanäle sind entsprechend aufgebaut.

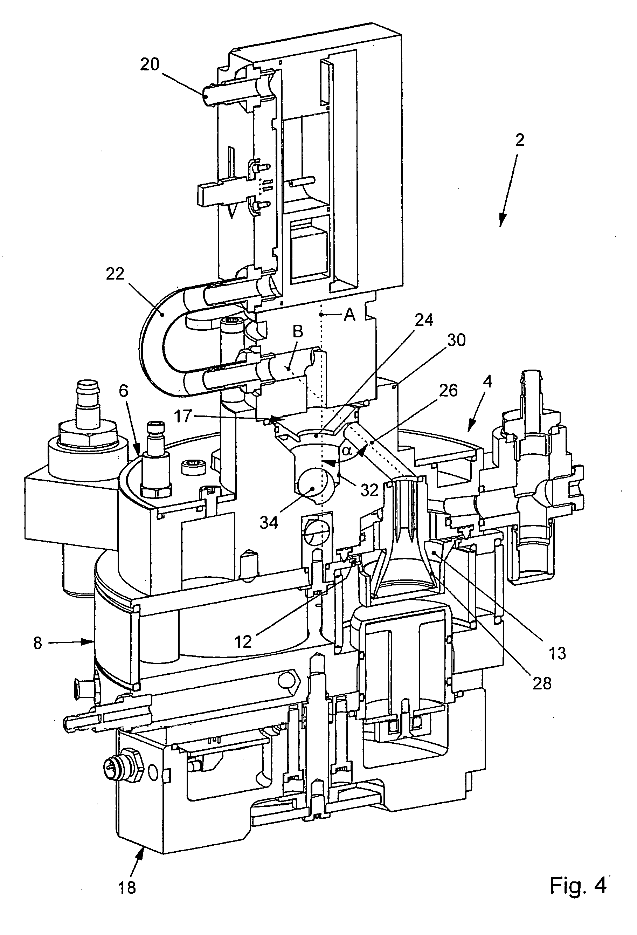

Bei dem in Figur 4 dargestellten Ausführungsbeispiel sind die Strömungskanäle in Umfangrichtung der Vorrichtung 2 zueinander versetzt angeordnet, nämlich entsprechend der um etwa 120° zueinander versetzten Anordnung der Aufnahmen 10, 12 und 14 um ebenfalls etwa 120°. Entsprechend den jeweiligen Anforderungen können erfindungsgemäss selbstverständlich auch lediglich zwei oder mehr als drei Aufnahmen und damit Kulturbehälter 10, 12, 14 sowie zugeordnete Strömungskanäle 26 vorgesehen sein.

Der Strömungskanal 26 verläuft von dem Verteilungsraum 24 radial nach aussen und geneigt nach unten, so dass die Strömung der Testatmosphäre ebenfalls radial nach aussen und nach unten verläuft. Um die Strömungsverhältnisse bei der Beaufschlagung einer Kultur zu verbessern, ist am auslassseitigen Ende des Strömungskanals 26 eine sich in Strömungsrichtung erweiternde Mündung 28 gebildet, die beispielsweise so ausgebildet ist, wie in der DE 102 11 324 A1 beschrieben.

Hier zeigt sich als erstes der intramodulare Aufbau des Aerosolführungs-Moduls 6. Die Mündungen 28 gehören zu auswechselbaren Aerosoldüsen, die auf Wunsch entsprechend ihrer Länge bzw. ihres Durchmessers ausgewählt werden können. In diesen Mündungen 28 können auch auswechselbare Gittereinsätze sitzen, die dann natürlich dem Durchmesser der Aerosoldüse mit oder ohne Blende angepasst sein können.

Zum intramodularen Aufbau des Aerosolführungs-Moduls 6 gehören ferner auch Schlauchadapter 9 oder Photometer und durch einen Verschlussstopfen 7 verschlossene Öffnungen zum Einsetzen von beispielsweise einem Temperatur- oder Feuchtefühler.

Wie aus den Figuren 1 und 3 ersichtlich ist, ist das Probenaufnahme-Modul 8 bei diesem Ausführungsbeispiel im wesentlichen rotationssymmetrisch begrenzt, wobei die Aufnahmen 10, 12, 14 für die Kulturbehälter entlang eines Kreises angeordnet sind, der zu der Rotationssymmetrieachse im wesentlichen koaxial ist. Bei dem dargestellten Ausführungsbeispiel sind die Aufnahmen 10, 12, 14 in Umfangsrichtung äquidistant zueinander angeordnet. Die drei Aufnahmen 10, 12 und 14 sind somit in Umfangsrichtung um jeweils 120° zueinander versetzt angeordnet.

Das Probenaufnahme-Modul 8 ist ebenfalls intramodular aufgebaut. Dies gilt vor allem bezüglich der auswechselbaren Kulturbehälter. Es sollen Zellkultureinsätze verschiedener Fabrikate und Grössen oder auch Petrischalen einsetzbar sein. Hierzu sind verschiedene Adapter für die verschiedenen Einsätze vorgesehen.

Die Funktionsweise der vorliegenden Erfindung ist folgende:
Bei Benutzung der Vorrichtung 2 strömt die Testatmosphäre, beispielsweise Tabakrauch, durch die Einlassöffnung 20 in das Vorbereitungs-Modul 16, wo in der Testatmosphäre enthaltene Partikel elektrostatisch aufgeladen werden. Von dem Vorbereitungs-Modul 16 strömt die Testatmosphäre in die Strömungsführung 22 und zu deren Verteilungsraum 24. Von dem Verteilungsraum 24 strömen Teilströmungen der Testatmosphäre durch die Strömungskanäle 26 zu den Kulturbehältern 11, 13, 15 und beaufschlagen in den Kulturbehältern aufgenommene Kulturen, beispielsweise Zell- oder Bakterienkulturen.

Eine Abscheidung von in der Testatmosphäre enthaltenen Partikeln auf den Kulturen wird dadurch gefördert, dass im Bereich der Kulturen durch ein Sockel-Modul 18 ein elektronisches Feld erzeugt wird. Dadurch, dass der Strömungsweg der Testatmosphäre von dem Verteilungsraum 24 zu den einzelnen Kulturbehältern 11, 13, 15 gleich lang ist, ist erfindungsgemäss sichergestellt, dass sich die Konzentration an Partikeln in der Testatmosphäre aufgrund unterschiedlich langer Strömungswege zu den einzelnen Kulturbehältern nicht in unterschiedlichem und die Messergebnisse verfälschendem Masse verändert.

Bei dem dargestellten Ausführungsbeispiel wird lediglich ein Teilstrom der Testatmosphäre über die Strömungskanäle 26 zu den Kulturbehältern 11, 13, 15 geleitet. Derjenige Teilstrom, der nicht zu den Kulturbehältern 11, 13, 15 geleitet wird, wird über eine in dem Strömungskanalblock 30 gebildete, mit dem Verteilungsraum 24 in Verbindung stehende axiale Bohrung 32 (vgl. Fig. 3) und eine mit der Bohrung 32 in Verbindung stehende radiale Bohrung 34 abgeleitet. Es ist erfindungsgemäss jedoch auch möglich, die gesamte Strömung der Testatmosphäre den Kulturbehältern 11, 13, 15 zuzuleiten.

Wie aus Figur 4 ersichtlich ist, verläuft bei dem dargestellten Ausführungsbeispiel eine Längsachse des bei diesem Ausführungsbeispiel rotationssymmetrisch ausgebildeten Verteilungsraumes 24, die durch eine strichpunktierte Linie A symbolisiert ist, zu einer durch eine gestrichelte Line B symbolisierten Längsachse des Strömungskanals 26 unter einem Winkel α, der wesentlich kleiner als 90° ist, nämlich bei dem dargestellten Ausführungsbeispiel etwa 45°. Auf diese Weise ergibt sich ein besonders gleichmässiger Strömungsverlauf, der nicht durch abrupte Übergänge oder Ecken in der Strömungsführung gestört ist. Dies gilt allerdings nicht, wenn, wie gezeigt, ein trichterförmiger Kondensatabscheider 17 vorgesehen ist.

Nachdem die Testatmosphäre die in den Kulturbehältern 11, 13, 15 aufgenommenen Zell- und/oder Bakterienkulturen überströmt hat, wird sie aus den Kulturbehältern 11, 13, 15 abgeleitet. Die Art und Weise der Abteilung ist jedoch im Kontext der Erfindung nicht weiter von Interesse und wird daher hier nicht näher erläutert.

Bei dem dargestellten Ausführungsbeispiel sind werkzeuglos lösbare Verbindungsmittel zum Verbinden des Vorbereitungs-Moduls 16 mit dem Aerosolführungs-Modul 6 vorgesehen. Die Verbindungsmittel weisen zwei Verriegelungs-Einrichtungen 36, 36' (vgl. Fig. 1) auf, wobei nachfolgend lediglich die Verriegelungs-Einrichtung 36 näher erläutert wird. Die Verriegelungs-Einrichtung 36' ist entsprechend aufgebaut. Das Verriegelungs-Modul 36 ist um eine vertikale Drehachse drehbar an dem Aerosolführungs-Modul gelagert und als zweiarmiger Hebel ausgebildet, dessen einer Hebelarm 38 als Betätigungsarm zum Verdrehen der Verriegelungs-Einrichtung 36 um die Drehachse ausgebildet ist und dessen anderer Hebelarm 40 einen Formschluss mit dem Vorbereitungs-Modul 16 herstellt. Hierzu weist das Vorbereitungs-Modul 16 eine umlaufende Ringnut 42 auf, in die der Hebelarm 40 in der Verbindungsposition eingreift (vgl. Fig. 1). Fig. 1 stellt das Vorbereitungs-Modul 16 in einer Position dar, in der es nicht mit dem Aerosolführungs-Modul 6 verbunden ist.

Zum Verbinden des Vorbereitungs-Moduls 16 mit dem Aerosolführungs-Modul 6 wird das Vorbereitungs-Modul 16 auf das Aerosolführungs-Modul 6 aufgesetzt. Daran anschliessend werden die Verriegelungs-Einrichtungen 36, 36' um ihre jeweilige Drehachse, die eine Gewindespindel ist, verdreht, so dass die Hebelarme 40, 40' in die Ringnut 42 eingreifen und gleichzeitig nach unten drücken, was einen Form- und Kraftschluss bewirkt. Die entsprechenden Gewindespindeln der Verriegelungs-Einrichtungen sind dementsprechend links- bzw. rechtsdrehend ausgebildet. Zum Lösen des Vorbereitungs-Moduls 16 von dem Grundkörper 4 werden die Verriegelungs-Module 36, 36' so verdreht, dass ihre Hebelarme 40, 40' aus der Ringnut 42 freikommen.

Fig. 2 stellt das Vorbereitungs-Modul 16 in einer Position dar, in der es mit dem Aerosolführungs-Modul 6 verbunden ist. Falls entsprechend den jeweiligen Anforderungen und gewünschten Untersuchungen erforderlich, kann das Vorbereitungs-Modul 16 auf schnelle und einfache Weise gegen ein anderes Vorbereitungs-Modul, beispielsweise ein Direktexpositionsmodul, ausgetauscht werden.

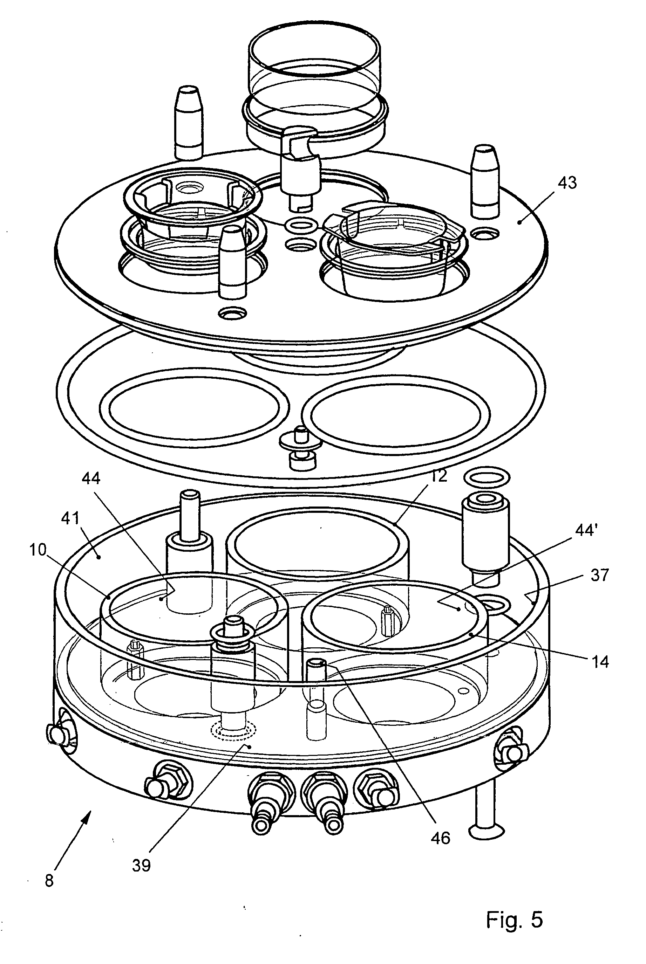

Wie aus Fig. 5 ersichtlich ist, weisen die Aufnahmen 10, 12, 14 bei diesem Ausführungsbeispiel jeweils eine kreisförmig begrenzte Aussenwandung auf und sind jeweils durch ein Glasrohr gebildet. Wie ferner ersichtlich, ist eine ebenfalls kreisförmig begrenzte Innenwandung 37 vorgesehen, die ebenfalls durch ein Glasrohr gebildet ist. Zwischen einem Boden 39 des Probenaufnahme-Moduls, den Aussenwandungen der Aufnahmen 10, 12, 14, der Innenwandung 37 und einem Deckel 43 des Probenaufnahme-Moduls 8 ist eine flüssigkeitsdichte Kammer 41 vorgesehen, die bei Benutzung der Vorrichtung 2 von einem Temperierfluid durchströmt wird, das durch eine Temperierflüssigkeit gebildet ist. Die Temperierflüssigkeit tritt durch zwei Einlässe 44, 44' in die Kammer 41 ein und durch ein bei diesem Ausführungsbeispiel als Überlauf ausgebildeten Auslass 46 aus der Kammer aus.

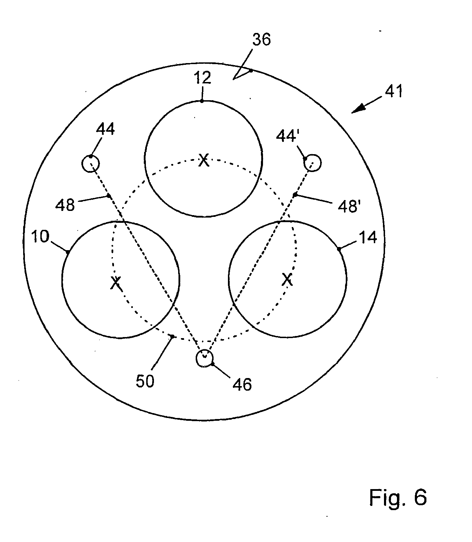

Fig. 6 zeigt stark schematisiert eine Draufsicht auf die Kammer 41, wobei ersichtlich ist, dass beispielsweise die Aufnahme 10 in einem zwischen dem Einlass 44 und dem Auslass 46 verlaufenden Strömungsweg der Temperierflüssigkeit angeordnet ist, so dass die Aufnahme 10 intensiv und gleichmässig von der Temperierflüssigkeit umströmt wird. Auf diese Weise ergibt sich eine besonders genaue und gleichmässige Temperierung der Aufnahme 10 und damit des in der Aufnahme 10 aufgenommenen Kulturbehälters 11. Entsprechendes gilt für die in den Aufnahmen 12 und 14 aufgenommenen Kulturbehälter 13 und 15. In Gebrauchslage sind diese im Übrigen bis zu einem Niveau unterhalb der Zellkulturen mit Nährlösung gefüllt und von dieser durch eine durchlässige Membrane getrennt.

Wie weiter ersichtlich ist, ist die Aufnahme 10 auf einer gedachten Verbindungslinie 48 zwischen dem Einlass 44 und dem Auslass 46 angeordnet. Entsprechendes gilt für die Aufnahme 14, die auf einer gedachten Verbindungslinie 48' zwischen dem Einlass 44' und dem Auslass 46 angeordnet ist.

Die Aufnahmen 10, 12, 14 sind bei diesem Ausführungsbeispiel entlang des Umfangs eines in Fig. 6 durch eine strichpunktierte Linie symbolisierten Kreises 50 angeordnet, der zu der im wesentlichen kreisförmig begrenzten Innenwandung 37 konzentrisch ist. Bei dem dargestellten Ausführungsbeispiel sind die Aufnahmen 10, 12, 14 in Umfangsrichtung des Kreises 50 im wesentlichen äquidistant zueinander angeordnet.

Die Erfindung ermöglicht somit eine genaue und einfache Temperierung der Kulturbehälter, um beispielsweise eine Kondensatbildung zu vermeiden. Auf diese Weise sind die Untersuchungsergebnisse bei der Untersuchung von Zell- bzw. Bakterienkulturen mit der erfindungsgemässen Kultur-/Expositionsvorrichtung verbessert.

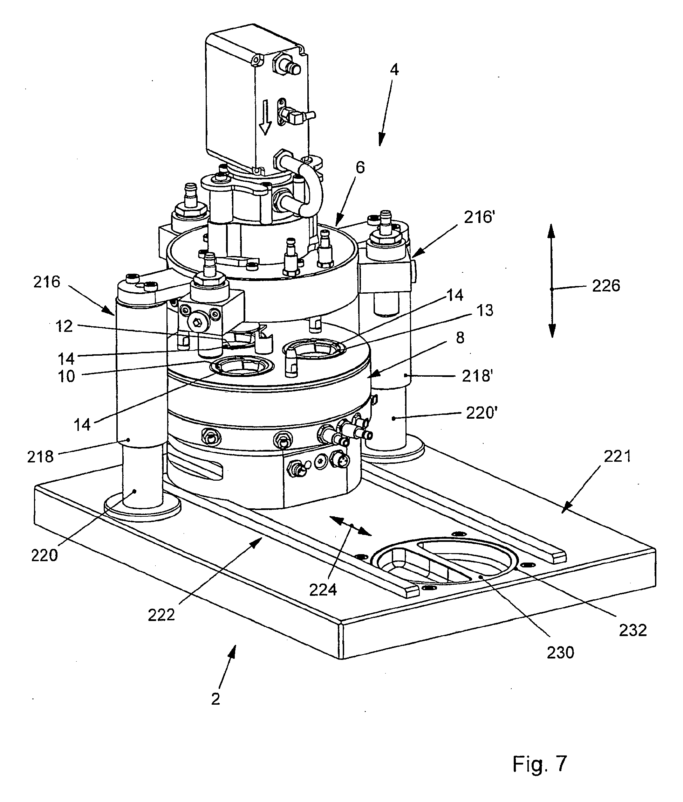

Nach einem weiteren Ausführungsbeispiel der Erfindung gemäss den Figuren 7 bis 10 erfolgt eine Bewegungsführung des Aerosolführungs-Moduls 6 relativ zum Probenaufnahme-Modul 8 mittels einer Führung, die dadurch gebildet ist, dass zwei erste Linearführungen 216, 216' das Aerosolführungs-Modul 6 vertikal führen. Die ersten Linearführungen 216, 216' sind jeweils durch zwei zylinderartige Körper 218, 218', 220, 220' gebildet, die zueinander teleskopartig angeordnet sind. Zur Realisierung der linearen Führung ist jeweils der zylinderartige Körper 218, 218' mit dem Aerosolführungs-Modul 6 verbunden und jeweils der zylinderartige Körper 220, 220' mit einer Halteplatte 221.

Sofern sich das Aerosolführungs-Modul 6 in einer Offenstellung befindet, können die Kulturbehälter 11, 13, 15 dem Probenaufnahme-Modul 8 entnommen werden. Dazu wird mittels einer zweiten Linearführung 222 das Probenaufnahme-Modul 8 in einer Bewegungsrichtung 224 geführt, die orthogonal zur Bewegungsrichtung 226 der ersten Linearführung angeordnet ist.

Zur Erzeugung einer Senkbewegung des Aerosolführungs-Moduls 6 über die erste Linearführung 216, 216' ist ein Getriebe 228 an der Unterseite der Halteplatte 221 angeordnet, das in den Figuren 7, 9 und 10 näher erläutert wird. Zur Erzeugung einer Eingangsbewegung sieht die Vorrichtung 2 ein Betätigungsmittel vor, welches durch eine handbetätigbare Drehscheibe 230 gebildet ist. Die handbetätigbare Drehscheibe 230 ist in einer Ausnehmung 232 der Halteplatte 221 angeordnet. Die kinematische Kette zwischen der Bewegung der Drehscheibe 230 und der ersten Linearführung 216, 216' wird nachfolgend näher erläutert.

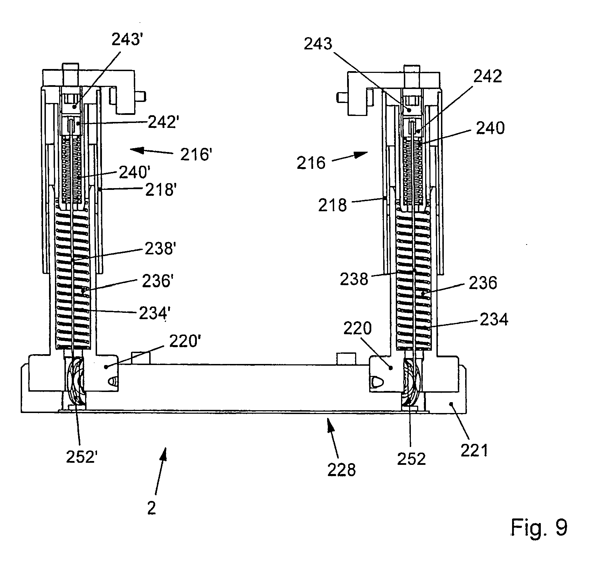

Die Verwendung einer Drehscheibe 230 in Kombination mit dem Getriebe 228 hat zum Vorteil, dass das Verhältnis zwischen der Kraft an Drahtseilen 238, 238' (vgl. Fig. 9) und dem Drehmoment der Drehscheibe 230 proportional zu 1/sin (Drehwinkel der Drehscheibe 230) ist, so dass insbesondere gegen Ende der Betätigung der Drehscheibe 230 (180°, Schliessstellung) sehr hohe Seilkräfte erzeugt werden können, was nützlich ist, da in dieser Situation die Dichtungen zwischen Aerosolführungs- und Probeaufnahme-Modul vorgespannt werden müssen.

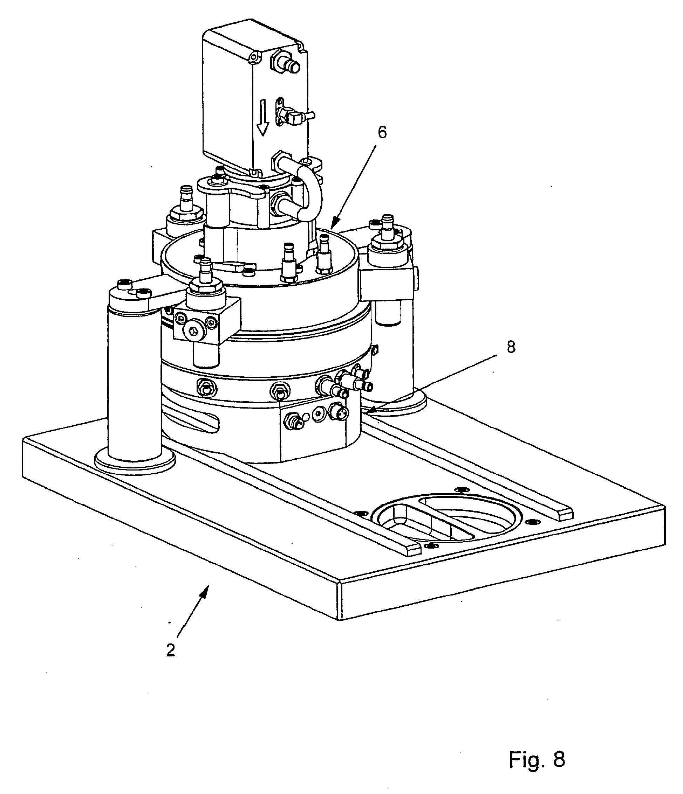

Fig. 8 zeigt das Ausführungsbeispiel Vorrichtung 2 in einer Schliessstellung, die erreicht wird, nachdem das Aerosolführungs-Modul 6 in einer Senkbewegung relativ zum Probenaufnahme-Modul 8 bewegt wurde. Das Aerosolführungs-Modul 6 ist in der Schliessstellung mit dem Probenaufnahme-Modul 8 dadurch strömungstechnisch verbunden.

Fig. 9 zeigt das Ausführungsbeispiel der Vorrichtung 2 in einem Vertikalschnitt gemäss Fig. 7, wobei die Darstellung auf die zum Verständnis wesentlichen Details reduziert ist.

Zur selbsttätigen Bewegung des Aerosolführungs-Moduls 6 (siehe Fig. 7) in die Offenstellung weist die erste Linearführung 216, 216' eine Feder 234, 234' auf, die als Spiralfeder geformt ist und in Schliessstellung des Aerosolführungs-Moduls 6 gespannt ist, so dass deren Federenergie zum Erreichen der Offenstellung in kinetische Energie des Aerosolführungs-Moduls 6 umgewandelt wird.

Die Feder 234, 234' ist im Inneren 236, 236' des zylinderartigen Körpers 220, 220' derart angeordnet, dass sie beim Absenken des zylinderartigen Körpers 218. gespannt wird. Dadurch wird erreicht, dass zum Erreichen der Offenstellung die Bewegung durch die Federkraft unterstützt wird.

An dem zylinderartigen Körper 218, 218' ist ein Drahtseil 238, 238' angeordnet, wodurch eine Eingangsbewegung in eine Antriebsbewegung für die erste Linearführung 216, 216' weitergeleitet wird. Die Umwandlung der Bewegung des Drahtseils 238, 238' erfolgt über das Getriebe 228, so dass die Eingangsbewegung, die über die Drehscheibe 230 erfolgt, in eine Antriebsbewegung für die ersten Linearführungen 216, 216' umgewandelt wird.

Zum Spannen des jeweiligen Drahtseiles 238, 238' dient jeweils eine zusätzliche Feder 240, 240', die im Inneren des zylinderartigen Körpers 218, 218' angeordnet ist. Sie dient ebenfalls dazu, mögliche ungewünschte Längenänderungen des jeweiligen Drahtseils 238, 238' auszugleichen. Die Befestigung des Drahtseils 238, 238' erfolgt über einen Haltekörper 242, 242', der an der jeweiligen zusätzlichen Feder 240, 240' angeordnet ist.

Die Haltekörper 242, 242' sind an Einstellschrauben 243, 243' angeordnet, die es einerseits wiederum ermöglichen, bei synchroner Vorstellung, die Vorspannung der Federn 240, '240' einzustellen und es andererseits ermöglichen, bei gegenläufiger Vorstellung eine Längendifferenz zwischen den Drahtseilen 238, 238' auszugleichen.

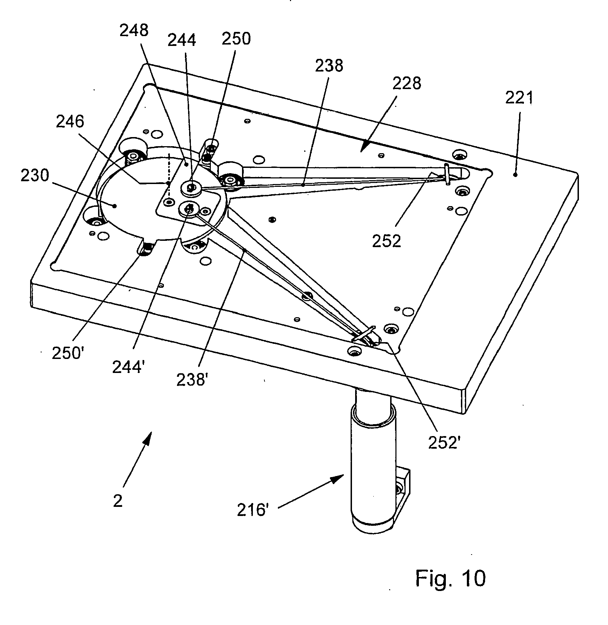

Fig. 10 zeigt die Kultur-/Expositionsvorrichtung in einer Perspektivansicht von der Unterseite. Ein Teil des Getriebes 228 ist an der Unterseite der Halteplatte 221 angeordnet. Zur Bewegungsumformung sind an der handbetätigbaren Drehscheibe 230 zwei Drahtseile 238, 238' mittels jeweils eines Befestigungspunktes 244, 244' angeordnet, von denen jeweils ein Drahtseil einer der ersten Linearführung 216, 216' zugeordnet ist. Durch Drehen der Drehscheibe 230 führen die Befestigungspunkte 244, 244' eine Kreisbewegung um eine Drehachse 246 der Drehscheibe 230 aus, wodurch sich die jeweilige Strecke vom Befestigungspunkt 244, 244' zu einer Führungsrolle 252, 252' verkürzt. Durch die konstante bzw. nahezu konstante Länge der Drahtseile 238, 238' wird die Streckenänderung in eine Senk- oder Hubbewegung der ersten Linearführungen 216, 216' umgewandelt. Dadurch ergibt eine Drehbewegung der Drehscheibe 230 ein Heben oder Senken des Aerosolführungs-Moduls 6. Zur Bewegungsführung weist die Drehscheibe 230 einen Anschlag 248 auf, der durch die an der Halteplatte 221 angeordneten Gegenanschläge 250, 250' die Bewegung der Befestigungspunkte 244, 244' begrenzt, wodurch ebenfalls die Bewegung der ersten Linearführungen 216, 216' und damit die Hubbewegung des Aerosolführungs-Moduls 6 begrenzt ist.

Eine weitere Begrenzung der Bewegung der Befestigungspunkte 244, 244' ist durch die Anordnung der Befestigungspunkte 244, 244' an der Drehscheibe 230 erreicht. Diese sind an der Drehscheibe 230 in der Art angeordnet, dass sich beim Erreichen der Offenstellung bzw. der Schliessstellung Totpunktlagen ergeben, die ein selbsttätiges Bewegen der Drehscheibe 230 verhindern, so dass eine Bewegung des Aerosolführungs-Moduls 6 aus der Offenstellung bzw. aus der Schliessstellung erst durch Betätigen der Drehscheibe 230 möglich ist.

In diesem Zusammenhang hat die Zusatzfeder 240, 240' neben der genannten Aufgabe ebenfalls folgende Aufgaben:
Damit sich die Drehscheibe 230 nicht ungewünscht aus den Totpunktlagen bewegt, wird die Drehscheibe 230 z.B. zum Halten der Schliessstellung über die Totpunktlage hinausgedreht, wodurch in Kombination mit dem Anschlag 248 und den Gegenanschlägen 244, 244' erreicht wird, dass die Drehscheibe 230 sich nicht selbsttätig zurückdrehen kann. Dies hat jedoch zur Folge, dass sich das Aerosolführungs-Modul 6 wiederum in Richtung der Offenstellung bewegen kann, wodurch sich die zwischen Aerosolführungs-Modul 6 und Probenaufnahme-Modul 8 befindliche Dichtung (nicht dargestellt) entspannt und sich ggf. ein zu grosser Spalt zwischen Aerosolführungs-Modul 6 und Probenaufnahme-Modul 8 bildet. Zur Verhinderung eines zu grossen Spaltes, der ggf. die Dichtwirkung schwächt bzw. aufhebt, dient die Zusatzfeder 240, 240' dazu, ein ungewolltes Bewegen des Aerosolführungs-Moduls 6 in Richtung der Offenstellung zu unterbinden bzw. zu verringern.

Die Zusatzfeder 240, 240' gleicht ferner Toleranzen aus, die andernfalls zur Sicherstellung einer strömungstechnischen Verbindung zwischen Aerosolführungs-Modul 6 und Probenaufnahme-Modul 8 aufwendig behoben werden müssten.

Zur Führung und Umwandlung der Bewegungsrichtung des Drahtseils 238, 238' weist das Getriebe 228 jeweils die Führungsrollen 252, 252' (siehe Fig. 9) auf. Dadurch wird die Drehbewegung der Drehscheibe 230 in eine lineare Bewegung für die Bewegung des Aerosolführungs-Moduls 6 umgewandelt, so dass unter Wirkung der ersten Linearführung 216, 216' das Aerosolführungs-Modul 6 eine Senkbewegung ausführen kann. Die Hubbewegung erfolgt, wie bereits beschrieben, nach Aufhebung der entsprechenden Totpunktlage durch initiales Drehen der Drehscheibe 230.

**Bezugszeichenliste**

| | | | | | |
|---|---|---|---|---|---|
| 1 | | 34 | radiale Bohrung | 238 | Drahtseil |
| 2 | Vorrichtung | 35 | | 240 | Feder |
| 3 | | 36 | Verriegelungs-Einrichtung | 242 | Haltekörper |
| 4 | Grundkörper | 37 | Innenwandung | 243 | Einstellschraube |
| 5 | | 38 | Hebelarm | 244 | Befestigungspunkt |
| 6 | Aerosolführungs-Modul | 39 | Boden | 246 | Drehachse |
| 7 | Verschlussstopfen | 40 | Hebelarm | 248 | Anschlag |
| 8 | Probenaufnahme-Modul | 41 | Kammer | 250 | Gegenanschlag |
| 9 | Schlauchadapter | 42 | Ringnut | 252 | Führungsrolle |
| 10 | Aufnahme | 43 | Deckel | | |
| 11 | Kulturbehälter | 44 | Einlass | | |
| 12 | Aufnahme | 46 | Auslass | | |
| 13 | Kulturbehälter | 48 | Verbindungslinie | | |
| 14 | Aufnahme | 50 | Kreis | | |
| 15 | Kulturbehälter | | | A | Linie |
| 16 | Vorbereitungs-Modul | | | B | Linie |
| 17 | Kondensatabscheider | | | | |
| 18 | Sockel-Modul | | | | |
| 19 | | | | | |
| 20 | Einlassöffnung | | | | |
| 21 | | | | | |
| 22 | Strömungsführung | 216 | Linearführung | | |
| 23 | | 218 | zylinderartiger Körper | | |
| 24 | Verteilungsraum | 220 | zylinderartiger Körper | | |
| 25 | | 221 | Halteplatte | | |
| 26 | Strömungskanal | 222 | Linearführung | | |
| 27 | | 224 | Bewegungsrichtung | | |
| 28 | Mündung | 226 | Bewegungsrichtung | | |
| 29 | | 228 | Getriebe | | |
| 30 | Strömungskanalblock | 230 | Drehscheibe | α | Winkel |
| 31 | | 232 | Ausnehmung | | |
| 32 | axial Bohrung | 234 | Feder | | |
| 33 | | 236 | Inneres | | |

## Patentansprüche

1. Kultur-/Expositionsvorrichtung, insbesondere für Zell- und/oder Bakterienkulturen, mit Aufnahmen (10, 12, 14) für Kulturbehälter (11, 13, 15) in einem Grundkörper (4) und mit einer einen Einlass (20) aufweisenden Strömungsführung (22) zum Beaufschlagen der Kulturbehälter (11, 13, 15) mit einer Testatmösphäre,
**dadurch gekennzeichnet,**
**dass** die Vorrichtung aus mehr als zwei Modulen (6, 8, 16,18) bestehend aus zumindest einem Probenaufnahme-Modul (8), einem Aerosolführungs-Modul (6), einem Vorbereitungs-Modul (16) oder/und einem Sockel-Modul (18) aufgebaut ist und diese jeweils voneinander lösbar und austauschbar miteinander verbunden sind.

2. Kultur-/Expositionsvorrichtung nach Anspruch 1, **dadurch gekennzeichnet, dass** die Strömungsführung (22) eine Vertellungsöffnung oder einen Vertellungsraum (24) aufweist, von der bzw. von dem Strömungskanäle (26) im wesentlichen gleicher Länge zu den einzelnen Kulturbehältern führen.

3. Kultur-/Expositionsvorrichtung nach Anspruch 1 oder 2, **dadurch gekennzeichnet, dass** die Aufnahme (10, 12, 14) in einer Kammer (41) angeordnet ist, die von einem Temperlerfluid durchströmbar ist, und wobei zum Zuführen des Temperierfluids ein Einlass (44, 44') und zum Abführen des Temperierfluids ein Auslass (46) vorgesehen ist, zwischen denen ein Strömungsweg für das Temperierfluid gebildet ist und die Aufnahme (10, 12, 14) in dem Strömungsweg angeordnet ist, derart, dass der Kulturbehälter (11, 13, 15) ein Strömungshindernis für das Temperlerfluld bildet und dass die Kammer (41) eine im wesentlichen kreisförmig begrenzte Innenwandung (37) aufweist.

4. Kultur-/Expositionsvorrichtung nach wenigstens einem der Ansprüche 1 bis 3, **dadurch gekennzeichnet, dass** der Einlass (20) an dem lösbar mit dem Aerosolführungs-Modul (6) verbundenen Vorbereitungs-Modul (16) gebildet ist.

5. Kultur-/Expositionsvorrichtung nach wenigstens einem der Ansprüche 1 bits 4, **gekennzeichnet durch** werkzeuglos lösbare Verriegelungs-Einrichtungen (36, 36') zum Verbinden des Vorbereitungs-Moduls (16) mit dem Aerosolführungs-Modul (6).

6. Kultur-/Expositionsvorrichtung nach Anspruch 5, **dadurch gekennzeichnet, dass** die Verriegelungs-Einrichtungen (36, 36') einen Formschluss zwischen dem Vorbereitungs-Modul (16) und dem Aerosolführungs-Modul (6) herstellen.

7. Kultur-/Expositionsvorrichtung nach einem der vorhergehenden Ansprüche 1 bis 6, **dadurch gekennzeichnet, dass** das Vorbereitungs-Modul (16) als Vorbereitungs-Modul zur elektrostatischen Aufladung von in der Testatmosphäre enthaltenen Partikeln ausgebildet ist,

8. Kultur-/Expositionsvorrichtung nach wenigstens einem der Ansprüche 1 bis 7, **dadurch gekennzeichnet, dass** das Sockel-Modul (18) zum Anlegen eines elektrostatischen Feldes lösbar mit dem Probenaufnahmen-Modul (8) verbunden ist.

9. Kultur-/Expositionsvorrichtung nach einem der vorhergehenden Ansprüche 1 bis 8, **dadurch gekennzeichnet, dass** der Grundkörper (4) das Aerosolführungs-Modul (6) und das Probenaufnahme-Modul (8) aufweist, wobei in dem Probenaufnahme-Modul (8) wenigstens eine der Aufnahmen (10, 12, 14) für einen Kulturbehälter (11, 13, 15) gebildet ist, und wobei in dem Aerosolführungs-Modul (6) wenigstens die Strömungsführung (22) zur Zuführung einer Strömung der Testatmosphäre zu dem Kulturbehälter (11, 13, 15) gebildet ist.

10. Kultur-/Expositionsvorrichtung nach einem der vorigen Ansprüche 1 bis 9, **dadurch gekennzeichnet, dass** das Aerosolführungs-Modul (6) und das Probenaufnahme-Modul (8) an einer Führung relativ zueinander geführt sind und durch eine Antriebseinrichtung zwischen einer Schliessstellung, in der die Strömungsführung (22) strömungstechnisch mit dem Kulturbehälter (11, 13, 15) in Verbindung steht, und einer Offenstellung, in der die Strömungsführung (22) strömungstechnisch von dem Kulturbehälter (11, 13, 15) getrennt ist, relativ zueinander bewegbar sind.

11. Kultur-/Expositionsvorrichtung nach Anspruch 10, **dadurch gekennzeichnet, dass** die Führung wenigstens eine erste Linearführung (216, 216') zur linearen Führung des Aerosolführungs-Moduls (6) und des Probenaufnahme-Moduls (8) relativ zueinander zwischen der Schliessstellung und der Offenstellung aufweist.

12. Kultur-/Expositionsvorrichtung nach Anspruch 10 und 11, **dadurch gekennzeichnet, dass** die Antriebseinrichtung wenigstens ein Getriebe (228) aufweist zur Umformung einer Eingangsbewegung in eine Antriebsbewegung zum relativen Bewegen des Aerosolführungs-Moduls (6) zum Probenaufnahme-Modul (8) und/oder zur Umformung einer Eingangsbewegung in eine Antriebsbewegung zum relativen Bewegen des Probenaufnahme-Moduls (8) zum Aerosolführungs-Modul (6).

13. Kultur-/Expositionsvorrichtung nach Anspruch 12, **dadurch gekennzeichnet, dass** das Getriebe (228) wenigstens ein sell-, band- oder kettenförmiges Zugmittel aufweist zur Weiterleitung der Eingangsbewegung als eine Antriebsbewegung zum relativen Bewegen des Aerosolführungs-Moduls (6) zum Probenaufnahme-Modul (8) und/oder zum relativen Bewegen des Probenaufnahme-Moduls (8) zum Aerosolführungs-Modul (6).

14. Kultur-/Expositionsvorrichtung nach Anspruch 12 oder 13, **dadurch gekennzeichnet, dass** das Getriebe (228) die Eingangsbewegung in eine Senkbewegung des Aerosolführungs-Moduls (6) zum Probenaufnahme-Modul (8) umformt, insbesondere in eine vertikale oder im wesentlichen vertikale Senkbewegung.

15. Kultur-/Expositionsvorrichtung nach einem der vorhergehenden Ansprüche 10 bis 14, **dadurch gekennzeichnet, dass** die Führung eine zweite Linearführung (222) aufweist zur linearen Führung des Probenaufnahme-Moduls (8) orthogonal bzw. im wesentlichen orthogonal zur Führungsrichtung der ersten Linearführung, insbesondere zur horizontalen oder im wesentlichen horizontalen Führung des Probenaufnahme-Moduls.

## Claims

1. Culture/exposure device, in particular for cell and/or bacteria cultures, having receptacles (10, 12, 14) for culture containers (11, 13, 15) in a base body (4) and having a flow guide (22), which has an inlet (20), for charging the culture containers (11, 13, 15) with a test atmosphere,
**characterized in that**
the device is constructed from more than two modules (6, 8, 16, 18) comprising at least a sample-receiving module (8), an aerosol guide module (6), a preparation module (16) and/or a base module (18) and these are each connected to one another detachably from one another and exchangeably.

2. Culture/exposure device according to claim 1, **characterized in that** the flow guide (22) has a distribution opening or a distribution chamber (24) from which flow channels (26) substantially of the same length lead to the individual culture containers.

3. Culture/exposure device according to claim 1 or 2, **characterized in that** the receptacle (10, 12, 14) is arranged in a chamber (41) through which a temperature-controlling fluid can flow, and wherein an inlet (44, 44') is provided for feeding in the temperature-controlling fluid and an outlet (46) is provided for removing the temperature-controlling fluid, between which a flow path for the temperature-controlling fluid is formed, and the receptacle (10, 12, 14) is arranged in the flow path such that the culture container (11, 13, 15) forms an flow obstacle for the temperature-controlling fluid, and **in that** the chamber (41) has a substantially circularly demarcated inner wall (37).

4. Culture/exposure device according to at least one of claims 1 to 3, **characterized in that** the inlet (20) is formed on the preparation module (16) connected detachably to the aerosol guide module (6).

5. Culture/exposure device according to at least one of claims 1 to 4, **characterized by** locking devices (36, 36'), which can be released without tools, for connecting the preparation module (16) to the aerosol guide module (6).

6. Culture/exposure device according to claim 5, **characterized in that** the locking devices (36, 36') establish positive locking between the preparation module (16) and the aerosol guide module (6).

7. Culture/exposure device according to one of the preceding claims 1 to 6, **characterized in that** the preparation module (16) is constructed as a preparation module for electrostatic charging of particles contained in the test atmosphere.

8. Culture/exposure device according to at least one of claims 1 to 7, **characterized in that** the base module (18) is connected detachably to the sample-receiving module (8) for application of an electrostatic field.

9. Culture/exposure device according to one of the preceding claims 1 to 8, **characterized in that** the base body (4) comprises the aerosol guide module (6) and the sample-receiving module (8), wherein at least one of the receptacles (10, 12, 14) for a culture container (11, 13, 15) is formed in the sample-receiving module (8), and wherein at least the flow guide (22) for feeding a flow of the test atmosphere to the culture container (11, 13, 15) is formed in the aerosol guide module (6).

10. Culture/exposure device according to one of the preceding claims 1 to 9, **characterized in that** the aerosol guide module (6) and the sample-receiving module (8) are guided on a guide relative to one another and can be moved by a drive device relative to one another between a closed position, in which the flow guide (22) is connected in terms of flow to the culture container (11, 13, 15), and an open position, in which the flow guide (22) is separated in terms of flow from the culture container (11, 13, 15).

11. Culture/exposure device according to claim 10, **characterized in that** the guide comprises at least a first linear guide (216, 216') for linear guiding of the aerosol guide module (6) and of the sample-receiving module (8) relative to one another between the closed position and the open position.

12. Culture/exposure device according to claim 10 and 11, **characterized in that** the drive device comprises at least one gearing mechanism (228) for conversion of an input movement into a drive movement for relative movement of the aerosol guide module (6) with respect to the sample-receiving module (8) and/or for conversion of an input movement into a drive movement for relative movement of the sample-receiving module (8) with respect to the aerosol guide module (6).

13. Culture/exposure device according to claim 12, **characterized in that** the gearing mechanism (228) comprises at least one cable-, belt- or chain-like traction means for transmission of the input movement as a drive movement for relative movement of the aerosol guide module (6) with respect to the sample-receiving module (8) and/or for relative movement of the sample-receiving module (8) with respect to the aerosol guide module (6).

14. Culture/exposure device according to claim 12 or 13, **characterized in that** the gearing mechanisms (228) converts the input movement into a lowering movement of the aerosol guide module (6) with respect to the sample-receiving module (8), in particular into a vertical or substantially vertical lowering movement.

15. Culture/exposure device according to one of the preceding claims 10 to 14, **characterized in that** the guide comprises a second linear guide (222) for linear guiding of the sample-receiving module (8) orthogonally or substantially orthogonally to the guiding direction of the first linear guide, in particular for horizontal or substantially horizontal guiding of the sample-receiving module.

## Revendications

1. Dispositif de culture et d'exposition, en particulier pour les cultures de cellules et/ou de bactéries, avec des moyens de réception (10, 12, 14) de récipients de culture (11, 13, 15) dans un corps de base (4) et avec un guide d'écoulement (22) présentant un orifice d'entrée (20) pour soumettre les récipients de culture (11, 13, 15) à une atmosphère d'essai,
**caractérisé en ce**
**que** le dispositif est constitué de plus de deux modules (6, 8, 16, 18) composés d'au moins un module de réception d'échantillons (8), d'un module de guidage d'aérosol (6), d'un module de préparation (16) et/ou d'un module de socle (18) et que ces derniers sont raccordés de manière amovible et échangeable l'un à l'autre.

2. Dispositif de culture et d'exposition selon la revendication 1, **caractérisé en ce que** le guide d'écoulement (22) présente un orifice de distribution ou une chambre de distribution (24) à partir duquel/de laquelle des canaux d'écoulement (26) sensiblement de même longueur mènent à des récipients de culture individuels.

3. Dispositif de culture et d'exposition selon la revendication 1 ou 2, **caractérisé en ce que** le moyen de réception (10, 12, 14) est disposé dans une chambre (41) qui peut être traversée par un fluide de régulation de température, et pour l'alimentation du fluide de régulation de température étant prévue une entrée (44, 44') et pour l'évacuation du fluide de régulation de température étant prévue une sortie (46), entre lesquelles est formé un chemin d'écoulement du fluide de régulation de température, et le moyen de réception (10, 12, 14) étant disposé dans le chemin d'écoulement de sorte que le récipient de culture (11, 13, 15) constitue un obstacle à l'écoulement du fluide de régulation de température et que la chambre (41) présente une paroi intérieure (37) délimitée de forme sensiblement circulaire.

4. Dispositif de culture et d'exposition selon au moins l'une des revendications 1 à 3, **caractérisé en ce que** l'entrée (20) est formée dans le module de préparation (16) raccordé de manière amovible au module de guidage d'aérosol (6).

5. Dispositif de culture et d'exposition selon au moins l'une des revendications 1 à 4, **caractérisé par** des moyens de verrouillage (36, 36') amovibles sans outils pour connecter le module de préparation (16) au module de guidage d'aérosol (6).

6. Dispositif de culture et d'exposition selon la revendication 5, **caractérisé en ce que** les moyens de verrouillage (30, 36') créent une liaison de forme entre le module de préparation (16) et le module de guidage d'aérosol (6).

7. Dispositif de culture et d'exposition selon l'une des revendications précédentes 1 à 6, **caractérisé en ce que** le module de préparation (16) est réalisé sous forme de module de préparation pour le chargement électrostatique de particules contenues dans l'atmosphère de test.

8. Dispositif de culture et d'exposition selon au moins l'une des revendications 1 à 7, **caractérisé en ce que** le module de socle (18) est, pour appliquer un champ électrostatique, raccordé de manière amovible au module de réception d'échantillons (8).

9. Dispositif de culture et d'exposition selon l'une des revendications précédentes 1 à 8, **caractérisé en ce que** le corps de base (4) présente le module de guidage d'aérosol (6) et le module de réception d'échantillons (8), dans le module de réception d'échantillons (6) étant réalisé au moins l'un des moyens de réception (10, 12, 14) d'un récipient de culture (15, 11, 13), et dans le module de guidage d'aérosol (6) étant formé au moins le guide d'écoulement (22) pour l'alimentation d'un flux de l'atmosphère de test vers le récipient de culture (11, 13, 15).

10. Dispositif de culture et d'exposition selon l'une des revendications précédentes 1 à 9, **caractérisé en ce que** le module de guidage d'aérosol (6) et le module de réception d'échantillons (8) sont guidés sur un guidage l'un par rapport à l'autre et sont déplaçables l'un par rapport à l'autre par un moyen d'entraînement entre une position de fermeture dans laquelle le guide d'écoulement (22) est, du point de vue technique d'écoulement, en communication avec le récipient de culture (11, 13,15) et une position ouverte dans laquelle le guide d'écoulement (22) est, du point de vue technique d'écoulement, séparé du récipient de culture (15, 11, 13).

11. Dispositif de culture et d'exposition selon la revendication 10, **caractérisé en ce que** le guide présente au moins un premier guide linéaire (216, 216') pour le guidage linéaire du module de guidage d'aérosol (6) et du module de réception d'échantillons (8) l'un par rapport à l'autre entre la position de fermeture et la position ouverte.

12. Dispositif de culture et d'exposition selon la revendication 10 ou 11, **caractérisé en ce que** le moyen d'entraînement présente au moins un engrenage (228) pour convertir un mouvement d'entrée en un mouvement d'entraînement pour le déplacement relatif du module de guidage d'aérosol (6) par rapport au module de réception d'échantillons (8) et/ou pour convertir un mouvement d'entrée en un mouvement d'entraînement pour le déplacement relatif du module de réception d'échantillons (8) par rapport au module de guidage d'aérosol (6).

13. Dispositif de culture et d'exposition selon la revendication 12, **caractérisé en ce que** l'engrenage (228) présente au moins un moyens de traction en forme de bande ou de chaîne pour la transmission du mouvement d'entrée comme mouvement d'entraînement pour le déplacement relatif du module de guidage d'aérosol (6) par rapport au module de réception d'échantillons (8) et/ou pour le déplacement relatif du module de réception d'échantillons (8) par rapport au module de guidage d'aérosol (6).

14. Dispositif de culture et d'exposition selon la revendication 12 ou 13, **caractérisé en ce que** l'engrenage (228) convertit le mouvement d'entrée en un mouvement de descente du module de guidage d'aérosol (6) par rapport au module de réception d'échantillons (8), en particulier en un mouvement de descente vertical ou sensiblement vertical.

15. Dispositif de culture et d'exposition selon l'une des revendications précédentes 10 à 14, **caractérisé en ce que** le guide présente un deuxième guide linéaire (222) pour le guidage linéaire du module de réception d'échantillons (8) de manière orthogonale ou sensiblement orthogonale à la direction de guidage du premier guide linéaire, en particulier pour le guidage horizontal ou sensiblement horizontal du module de réception d'échantillons.
